# EUROPEAN PATENT APPLICATION

(11) **EP 2 575 113 A1**
(43) Date of publication of application: **03.04.2013**
(21) Application number: 11462018.0
(22) Date of filing: 30.09.2011
(51) Int. Cl.: G08B 21/04, A61B 5/00, G08B 29/18

(54) **Method and device for fall detection and a system comprising such device**

(71) Applicant: General Electric Company, Schenectady, NY 12345 (US)
(72) Inventor: Balázs, Jenö, 2040 Budaörs (HU); Erdös, Ákos, 2040 Budaörs (HU)
(74) Representative: Köteles, Zoltan

(57) **Abstract**

A method and a device for fall detection to be used in a health monitoring system in an in-house room environment are provided. The device comprises:
- a PIR detection unit **(10),** having a given field of view, and mounted on a vertical surface, and comprising
- a dual element PIR sensor **(12, 14),**
- an infrared lens placed in front of the PIR sensor **(12, 14)** and dividing a plane orthogonal to its axis of symmetry into a plurality of zones in the given field of view.

Each zone comprises two different sectors. The infrared lens is a Fresnel lens array **(11, 13)** having an optical focusing effect in a direction parallel with the axis of symmetry of the corresponding dual element PIR sensor **(12, 14),** which have orthogonal axis of symmetry. Output signals of both PIR sensors **(12, 14)** are forwarded to a signal processing MCU for evaluation of a phase of the output signals of the dual element PIR sensors **(12, 14)** and producing a fall detection alert signal in response to evaluation by the signal processing MCU.

## Description

### FIELD OF THE SUBJECT MATTER

The subject matter generally relates to a method and device for fall detection.

### BACKGROUND

More people rush to U.S. emergency rooms for injuries related to falling than from any other cause. And, according to the American Academy of Family Physicians, they're the primary cause of accidental death in people over the age of 65.

Not surprisingly, as we age the injuries we sustain from falls become more severe, which is why falls are the cause of 70 percent of accidental deaths in people aged 75 years and older.

Here are some other startling facts about falling from the USA Centers for Disease Control and Prevention (http://www.cdc.gov):
● One out of three adults age 65 and older falls each year.
● Among those age 65 and older, falls are the leading cause of injury death. They are also the most common cause of nonfatal injuries and hospital admissions for trauma.

● In 2007, over 18,000 older adults died from unintentional fall injuries.
● The death rates from falls among older men and women have risen sharply over the past decade.
● In 2009, 2.2 million nonfatal fall injuries among older adults were treated in emergency departments and more than 581,000 of these patients were hospitalized.
● In 2000, direct medical costs of falls totaled a little over $19 billion-$179 million for fatal falls and $19 billion for nonfatal fall injuries.

Different fall detectors are available. Some detector measures acceleration and body direction and is attached to a belt of the person. But people refusing or forgetting to wear this kind of detectors, still need a way to get help if they are incapable of getting up after a fall. Other sophisticated systems use IR camera and special image analyzes algorithms as described for e.g. in US Patent No. 7,541,934 entitled "Method and device for fall prevention and detection". Other methods use special short-range radars. In these cases the main issue is that several elderly people did not want to have installed such sophisticated systems in their home and environment, and also these systems are not cheep.

As described in the following publication (S. Brownsell and M. Hawley: Automatic fall detectors and the fear of falling. Journal of telemedicine and telecare, 10(5):262, 2004.) myriads of research has been done to detect the falls using wearable sensors, but the problem with this devices that the elderly people often forget to wear them.

Thus there is a need for a fall detection apparatus that is cheep and is accepted by the elderly people.

The PIR (Passive Infrared) sensors are largely used in burglar detection devices and so the elderly peoples are familiar with these devices to protect their property and are largely accepted.

### BRIEF SUMMARY

In accordance to one embodiment, a fall detection

device is provided for providing fall detection to be used in a health monitoring system in an in-house room environment, where the device comprising
- PIR (Passive Infra Red) detection unit, having a given field of view, and mounted on vertical surface, the PIR detection unit comprising
   - a dual element PIR sensor having an axis of symmetry,
   - infrared lens placed in front of the PIR sensor,
where the infrared lens is dividing a plane orthogonal to the axis of symmetry into several zones in the field of view of the PIR detection unit, each zone comprising two different sectors. The infrared lens is a Fresnel lens array having optical focusing effect in direction parallel with the axis of symmetry of the corresponding PIR sensor; and the PIR detection unit has two dual element PIR sensors of orthogonal axis of symmetry; and the output signals of both PIR sensors are forwarded to a subsequent signal processing MCU evaluating the output signals of the PIR sensors at least in their phases and producing fall detection alert signal in response to the result of said evaluation.

In accordance to another embodiment, a method is provided for providing fall detection signal to be used in a health monitoring system in an in-house room environment comprising
- PIR detection unit, having a given field of view, and mounted on vertical surface, the PIR detection unit comprising
   - a dual element PIR sensor having an axis of symmetry,
   - infrared lens placed in front of the PIR sensor,
   where the infrared lens is dividing a plane orthogonal to the axis of symmetry into several zones in the field of view of the PIR detection unit, each zone comprising two different sectors. The method comprises the steps of
   - placing an infrared Fresnel lens array in front of the corresponding PIR sensor, which Fresnel lens array has optical focusing effect in direction parallel with the axis of symmetry of the corresponding PIR sensor;
   - applying a PIR detection unit, comprising two dual element PIR sensors of orthogonal axis of symmetry;
   - forwarding output signals of both PIR sensors to a subsequent signal processing MCU;
   - evaluating the output signals of the PIR sensors at least in their phases and producing fall detection alert signal in response to the result of said evaluation.

In another embodiment, a system is provided which comprises at least one or a plurality of said devices for providing fall detection. A control unit comprising a user interface is connected to the at least one said device through communication interfaces, and the control unit is capable to establish real-time communication between a caregiver person and a person being in a supervised in-house room environment.

### BRIEF DESCRIPTION OF THE DRAWINGS

The subject matter will now be described in detail with reference to the accompanying drawings, in which
**Fig. 1** is a field of view of an embodiment of a horizontal movement detector in top view in accordance to the subject matter described herein,
**Fig. 2** is a field of view of a an embodiment of a horizontal movement detector in side view in accordance to the subject matter described herein,
**Fig. 3** is a field of view an embodiment of a vertical movement detection Fresnel lens in top view in accordance to the subject matter described herein,
**Fig. 4** a field of view of an embodiment of a vertical movement detection PIR sensor and Fresnel lens combination in side view in accordance to the subject matter described herein,
**Fig. 5** is a schematic arrangement of two PIR sensors in a fall detection unit in accordance to the subject matter described herein,
**Fig. 6** illustrates the signals delivered by the horizontal and vertical movement sensing PIR sensors in a fall detection unit in accordance to the subject matter described herein,
**Fig. 7** the block diagram of an embodiment of an analog signal processing unit and digital signal processing MCU in a fall detection unit in accordance to the subject matter described herein,
**Fig. 8** is a block diagram of another embodiment of the fall detection unit according to the invention utilizing a mixed signal processing unit,
**Fig. 9** shows in side view the detection of a standing/lying person by a fall detection unit according to an embodiment of the subject matter described herein with dual sensor arrangement,
**Fig. 10** is a block diagram of another embodiment of the fall detection unit in accordance to the subject matter described herein utilizing a mixed signal processing unit, with triple sensor arrangement,
**Fig. 11** shows in side view the detection of a standing/lying person by a fall detection unit in accordance to the subject matter described herein with triple sensor arrangement,
**Fig. 12** is a flow-chart of extended sensing algorithms by a fall detection unit according to an embodiment of the subject matter described herein, with triple sensor arrangement,
**Fig. 13** is a block diagram of a fall detection system in accordance to the subject matter described herein,
**Fig. 14** is a flow-chart of fall detection by a fall detection unit and system in accordance to the subject matter described herein.

### DETAILED DESCRIPTION

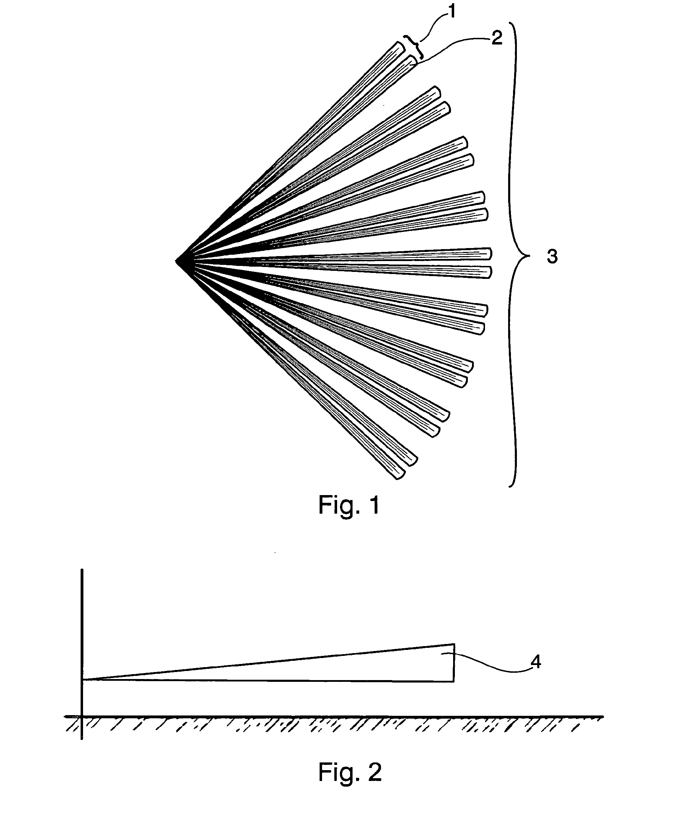

Fig. 1 shows a top field of view of one embodiment of a horizontal movement detector comprising Fresnel lens array. A field of view **3** has been divided into a plurality of detection zones **1.** One zone **1** comprises two sectors **2** which is created due to the fact that the PIR sensor of the sensor what we use is of a dual type one. The angle of field of view 3 of the horizontal movement detector is mainly depending on the type of used PIR sensor. For available PIR sensors on the market this angle is between 80° and 110°. Fresnel lenses in front of dual element PIR sensors are used to detect the horizontal movements divide the horizontal plane in several, for e.g. 9 or more zones **1** within the field of view **3** of a horizontal PIR sensor. Each zone **1** comprises two different sectors **2.** Movement of a person across a zone **1,** i.e. its sectors **2,** situated in said field of view **3** of the horizontal PIR will result sudden changes in the amount of IR radiation and a signal will be generated at the output of the corresponding PIR sensor. The signal phase is directly depending on the horizontal movement direction component of the person - left to right or vice versa.

**Fig. 2** shows the side view of a horizontal movement detection field of view structure of a detector comprising Fresnel lens array. We can see here just one zone **4.** In this way the horizontal movement detector is not sensitive to the vertical movements.

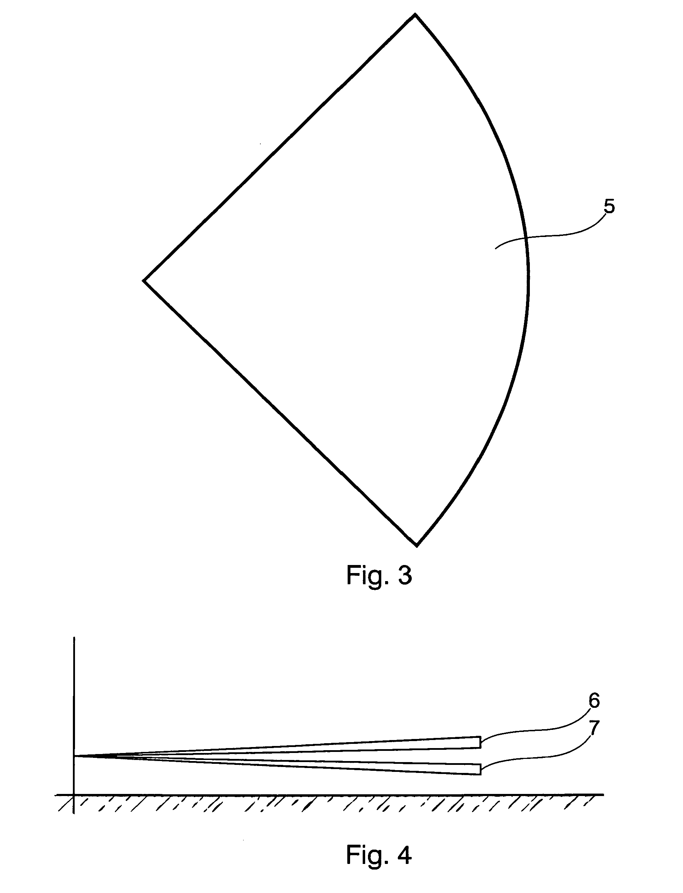

The second PIR sensor is rotated by 90 degrees, relatively to the horizontal PIR sensor, thus it has the ability to detect just vertical movements. In **Fig. 3** the top view of the field of view for the vertical movement detection Fresnel lens is represented. The second PIR sensor and Fresnel lens combination has a single horizontal sector **5.** In **Fig. 4** a field of view of a vertical movement detection PIR sensor and Fresnel lens combination is illustrated in side view. Here one detection zone is formed with two sectors **6** and **7.** The vertical movements, which have the main importance for us, are just under the installation plane of the sensor. To fulfill this requirement, a Fresnel lens array, which has a so called horizontal barrier allay, with a very sharp filed of view, is placed in front of this PIR sensor. The magnitude of this angle is dependent on the maximum distance what we want to survey and the height of the PIR sensor relative to the floor.

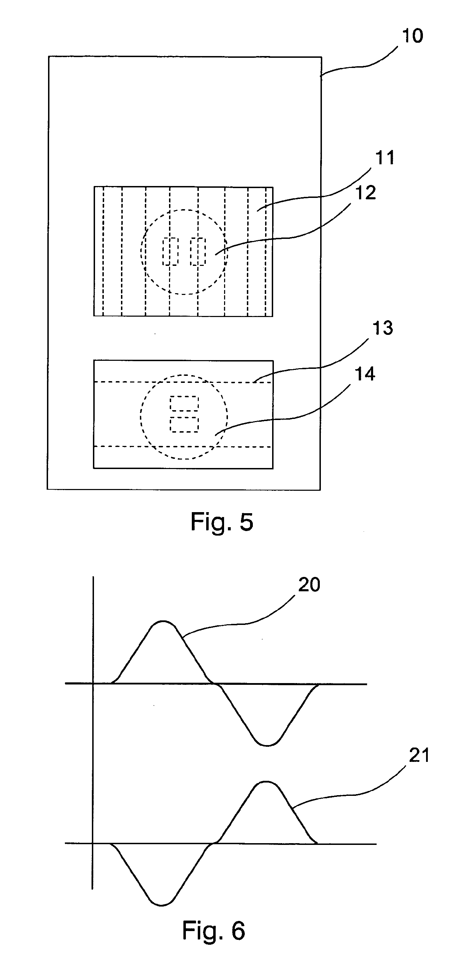

The schematic arrangement of both PIR sensors in the fall detection device is shown in **Fig. 5****.** Here is a PIR detection unit **10** is presented. A horizontal Fresnel lens **11** and a horizontal PIR sensor **12** are arranged for the movement detection. There is an array of vertical Fresnel lens **13** used for the vertical movement detection. Also there is a vertical PIR sensor **14** for vertical movement detection.

A PIR detection unit **10** according to the subject matter comprises a dual element PIR sensor **12, 14** having an axis of symmetry, and an infrared lens placed in front of those. The infrared lens divides a plane orthogonal to the axis of symmetry into several zones **1** in the field of view **3** of the PIR detection unit **10.** Each zone **1** comprises two different sectors **2.** The infrared lens is an array of Fresnel lenses **11, 13** having optical focusing effect in direction parallel with the axis of symmetry of the corresponding PIR sensor **12, 14.** The two dual element PIR sensors **12, 14** have orthogonal axis of symmetry. The output signals of both PIR sensors **12, 14** are forwarded to a subsequent signal processing MCU **35** evaluating the output signals of the PIR sensors **12, 14** at least in their phases and producing fall detection alert signal in response to the result of said evaluation.

The horizontal PIR sensor **12** may have in an embodiment vertical axis of symmetry, and the vertical PIR sensor **14** may have horizontal axis of symmetry.

The horizontal PIR sensor **12** in an embodiment may have zones **4** narrower than 10°.

The PIR detection unit **10** in an embodiment is mounted on a vertical surface at a position in which the vertical PIR sensor **14** is in 0.4 - 0.9 m distance from the floor.

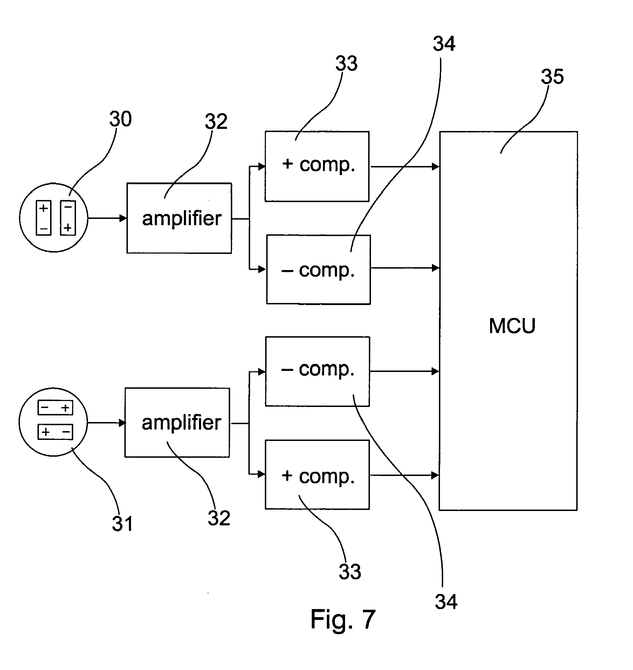

**Fig. 6** illustrates the signals delivered by the movement sensing horizontal and vertical PIR sensors **12** and **14.** A signal **20** is the one coming from the PIR sensor in case of right to left or down to top up movement, while a signal **21** is the one coming from the PIR sensor in case of left to right or up to down movement. These signals **20, 21** will be amplified, according to **Fig. 7****,** in addition a band pass filter can be utilized to filter out signals in undesired frequency range and the phase of the signal is detected by a positive comparator **33** and negative comparator **34.** Similar process is valid for the vertical movement detection.

The output signals of the amplifiers **32** are fed into comparators **33** and **34** which are connected to the interrupt input of the MCU **35.** Instead of the comparators **33** and **34** the signal could be fed into an analog digital converter, however this would cause the MCU consume more power, thus this solution is less practical in a device where low power consumption would be necessary.

In the schematic circuit arrangement of **Fig. 7** a signal processing MCU **35** receives four signals from four comparators **33, 34.** Two signals are coming from the horizontal movement detector, the PIR sensor **30,** through an amplifier **32** where these two signals are used to detect a horizontal movement. The amplifier **32** may comprise a band pass filter. The movement will be validly detected if the negative signal is followed, in a predefined time frame, by a positive one or vice versa. This predefined time frame can be, for example, a value between 1 to 10 seconds. This is important to reduce the eventual noises collected by PIR sensor **30** or coming from the amplifier **32.** In addition, two signals are similarly coming from the vertical movement detection subsystem, the PIR sensor **31,** through an amplifier **32.** In the same manner, as at the horizontal movement detection, the movement will be validly detected if a negative signal follows the positive signal in a predetermined time frame or vice versa. In additional the direction of the movement is detected at the vertical movement as well as at the horizontal movement.

The role of the band pass filter is to eliminate the DC current component and to filter out the noises from the signal. The amplified and filtered signal will be delivered to a positive signal comparator **33** and the negative signal comparator **34.** The output of the negative and positive signal comparator are connected to a signal processing unit, the MCU **35.**

The MCU **35** may contain an algorithm that is able to process the data received from the PIR sensors **30, 31,** and able to send an alert message via a communication link. The connection can be wired or wireless depending on the realization of the sensor.

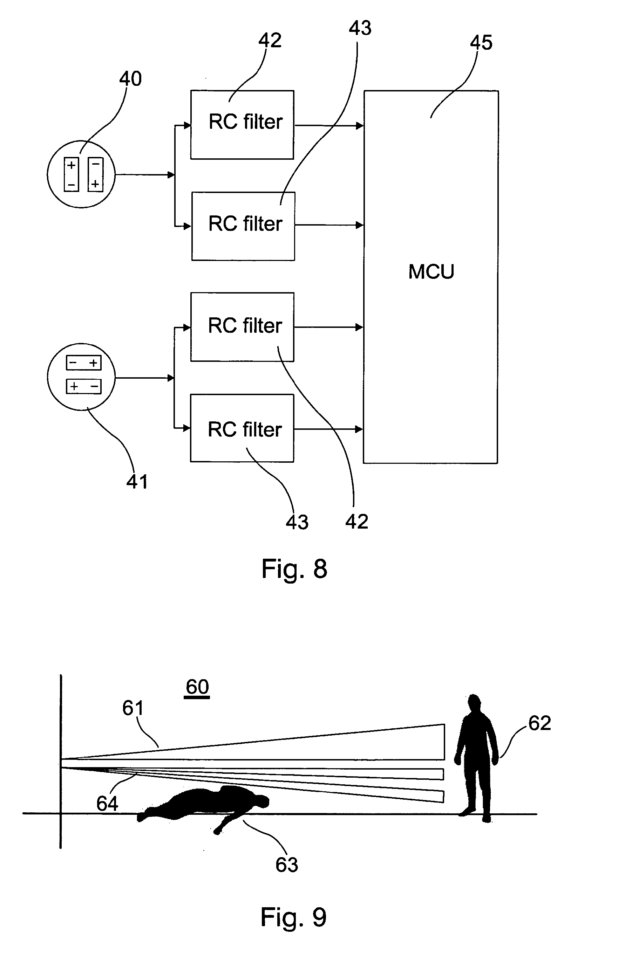

In the **Fig. 8** is representing another possible way to realize the signal processing from the dual PIR sensors. In this case the PIR sensors are connected to a mixed signal MCU **45** through two RC filters **42** and **43.** One of the filters **43** has the role to integrate the signal coming from the PIR sensors. The other filter **42** has the role of a low pass filter. The mixed signal digital MCU **45** has the role to detect the movements through special algorithms. This solution has the advantage that the raw signal from the PIR detectors **40** and **41** is fed directly to the input of MCU **45.** In this way further signal processing algorithms can be implemented within the MCU **45** in order to have a more precise detection of the vertical movements. This solution has the advantage that the row data coming from the PIR detectors **40** and **41** can be analyzed in more sophisticated way by the MCU **45.**

For a reliable sensing the horizontal sensor must be advantageously placed in a height where it is able to oversee each piece of seating in a room, and this view is not covered by any higher pieces of furniture. **Fig. 9** illustrates a possible sensor configuration **60** placed onto a wall of a room, where the sensors are at about the hip level of a standing person **62.** With this sensor setting the corresponding sensors have a horizontal zone **61** and a vertical zone **64** including two split sectors. This arrangement will not detect motion when the supervised person has already been lying on the floor, i.e. a lying person **63** will not be alone identified.

The detection algorithm running on the MCU **45** must be able to differentiate several motion patterns from a fall. For example, a person
1) bends down or
2) get on knees to pick up an object from the floor, or cleans something,
3) climbs down from a ladder,
4) walks down on stairway,
5) sits down on a chair,
6) lies down on a sofa or bed.

In order to differentiate these motion patterns from a fall can be very challenging, since a fall can have different characteristics. It can be a fast or a slow fall, moreover it can start either from standing position or from sitting or other posture, where the person is already being initially in a lower position.

In cases 1)-4) there is a high probability that the person is going to move in horizontal direction after a vertical movement, and while the vertical movement considerable amount of horizontal components of movement is present as well. In case of 5) the vertical movement can be presented with a corresponding low frequency, and most probably followed by detected horizontal movements as the person moves. Depending on the relative position of the outstanding sensor unit and the chair/sofa, it might be possible that only the head of the person will be visible (detectable) by the sensor. In case of 6) the low frequency vertical motion might not be followed by a horizontal motion if the furniture covers the whole body of the person. It shows the chance to confuse this event with a fall event, since in certain cases fall might have a relatively low frequency vertical component.

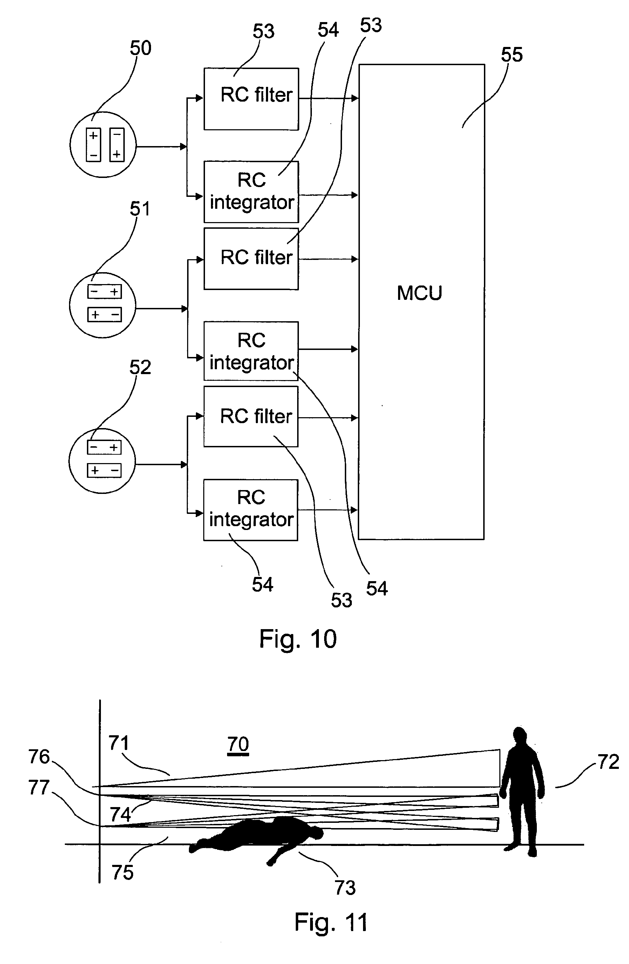

The circuit structure represented in **Fig. 10** makes possible to capture a continuous amplitude analog signal indicating the strength of the received signal. The block diagram illustrates this embodiment of the fall detecting PIR detection unit **10** using the signal from another additional vertical movements detecting second vertical PIR sensor **77** placed at a convenient vertical distance from the first vertical PIR sensor **76.** There is a movement detection horizontal PIR sensor **50** which is the same like shown earlier.

A first vertical PIR sensor **76** and a second vertical PIR sensor **77** together provide a sensor configuration **70** shown in **Fig. 11****.** The processing of signals derived from the first vertical PIR sensor **76** and the second vertical PIR sensor **77** is similar to the case of the embodiment presented in **Fig. 8****,** where raw signals from the PIR sensors **50, 51** and **52** are connected to a low pass RC filter **53** and to an RC integrator **54.** The preconditioned signals are fed to the input of the mixed signal processing MCU **55.**

The second vertical PIR sensor **77** may be arranged within the PIR detection unit or alternatively as a distinct unit. Setting a sampling rate about 300Hz and 5-8 bits resolution makes possible to obtain a reasonably reliable data.

In addition to the basic fall detection capabilities of the PIR sensors **50, 51, 76, 77** those will be able to provide other interesting information that can be useful at monitoring a person at home environment. The algorithms that help differentiating the fall events from other human motion patterns are able to detect motions from normal daily activity like sit down, lie down, walk, and so on, with a certain probability. This information might be transferred to a monitoring server, where that is stored and further algorithms might use it to make reasoning about the health condition of the monitored person, or it might be presented directly to the monitoring personals.

If the motion occurs in a larger distance from the sensor **50, 51, 76 or 77,** the frequency of the signal detected by the motion sensor **50, 51, 76 or 77** will be lower while the time period of the pattern of fall detection remains the same. Furthermore the infrared signal amplitude decreases with distance from the motion sensor **50, 51, 76 or 77.**

The MCU **55** may be configured to compare the time period, amplitude and frequency with one or more predetermined fall event patterns, to be able to classify the detected motion in order to be able to differentiate fall events from non-fall events.

The MCU **55** may be configured to differentiate between different fall patterns in order to determine the seriousness of the fall.

In another embodiment the device stores patterns from detected sensor data of previous fall events in memory, which fall events has already been confirmed by the user or caregiver. History of fall event patterns may be learnt with appropriate machine learning algorithms, and the learnt model is applied to perform a more reliable classification of further fall events.

As shown in **Fig. 11** extended sensing algorithms are possible if the device containing a third sensor, which is a second vertical PIR sensor **77** in addition to the basic first vertical PIR sensor **76,** that has a view on the lower field of the area to be monitored. The horizontal zone **71** is the same as horizontal zone **61** in sensor configuration **60** of **Fig. 9****.** In this case, however, the vertical detection is divided to two different zones **74, 75** where the upper zone **74** is detecting vertical motion at hip level by the first vertical PIR sensor **76,** and the lower zone **75** detecting motion below about an upright human hip level by the second vertical PIR sensor **77.** The different zones **74, 75** may be intersecting each other in a certain horizontal distance.

The main purpose of using the second vertical PIR sensor **77** placed in a lower position is to be able to identify the time range of detection of downward direction vertical motion signal after the upper first vertical PIR sensor **76,** is already stopped detecting motion. If the value of this time range exceeds a certain threshold level, indicates that the moving person probably had a fall. With this sensor arrangement the detection of whether the motion of a person had got to ground can be achieved with a greater confidence.

Properly arranged second vertical motion detector zone **75** gives the opportunity to detect whether the person is still moving after the fall. For this purpose the upper zone **74** shall be arranged that way that it cannot detect a moving person, which is lying on the floor, but the lower zone **75** must be able to partially detect a lying person **73.** **Fig. 11** l accordingly represents the detection of a standing person **72** and a lying person 73 by a fall detection unit according to an embodiment of the present subject matter with triple sensor arrangement.

The MCU may be configured that way, that as far after the fall the lower second vertical PIR sensor **77** detects motion, but the upper first vertical PIR sensor **76** does not, additional information to the generated alert signal is added that indicates to upper described circumstances.

With the 3 sensor arrangement where two PIR sensors are utilized to detect boundary crossing in vertical direction, it is possible to partially compensate the effects of sensing in different distance from the sensor.

Generally the method of the subject matter comprises the following steps:
- placing infrared Fresnel lenses **11, 13** in an array in front of the corresponding PIR sensor **12, 14,** which Fresnel lenses **11, 13** have optical focusing effect in direction parallel with the axis of symmetry of the corresponding PIR sensor **12, 14;**
- applying a PIR detection unit **10,** comprising two dual element PIR sensors **12, 14** of orthogonal axis of symmetry;
- forwarding output signals of both PIR sensors **12, 14** to a subsequent signal processing MCU **35;**
- evaluating the output signals of the PIR sensors **12, 14** at least in their phases and producing fall detection alert signal in response to the result of said evaluation.
The evaluating according to an embodiment can be a counter phase detecting within a predetermined time frame. The predetermined time frame can be an adjustable value between for e.g. 1 to 30 seconds.

In an embodiment the fall is detected when a detection of an up to down movement of a person is not followed by a down to up or horizontal movement detection in said predetermined time frame.

In an embodiment where a first vertical PIR sensor **76** and also a second vertical PIR sensor **77** are used, the output signals of the PIR sensors **76** and **77** are used together for evaluating and producing fall detection alert signal in response to the result of said evaluation.

Additional information about the fall can be gained by determining the speed of vertical downward motion. The following publication, (Wireless Sensor Networks for Activity Monitoring using Multi-sensor Multi-modal Node Architecture; Peter Hungt, Muhammad Tahir, Ronan Farrell, Sean McLoone and Tim McCarthy; Department of Electronic Engineering; Institute of Microelectronics and Wireless Systems; National University of Ireland Maynooth; Maynooth, Co. Kildare, Ireland) describes a multi-modal sensor node for human and vehicle activity monitoring, and proposes a method to achieve a more reliable direction and motion speed detection with 2 dual PIR sensors.

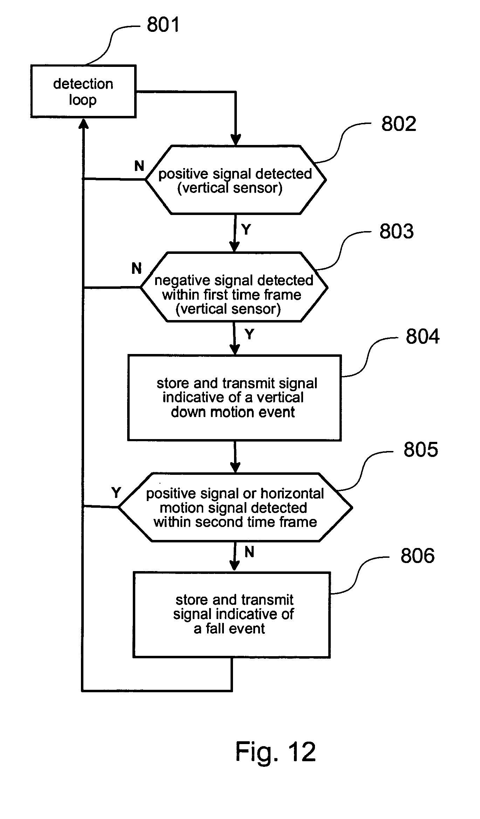

With reference to **Fig. 12** an exemplary algorithm has been illustrated for the dual motion sensing PIR detection unit **10.** MCU receives the signals from the comparators respective to the direction, and stores the direction data for further processing. In case of a fall the output of the vertical sensor will generate a negative signal **21** (see **Fig. 6****)** before the positive signal **20** which indicates that the movement direction is from up to down. At the beginning of the detection loop **801** in step **802** the MCU watches for a positive signal **20** detected from the output of vertical PIR sensor **31** of **Fig. 7****.** In the step **803** the MCU checks whether a positive signal detected, followed by a negative signal within a predefined time frame. If yes, in step **804** MCU stores and transmits a signal indicative of a vertical down motion event. In step **805** MCU checks whether the signal indicative of a downwards motion direction (vertical down motion event) is not followed by a signal generated by vertical movement from down to up or a horizontal movement within a predefined time frame, in step **806** the MCU generates an alert signal indicative of a fall event due to the fact that a fall of the person was detected with very high probability.

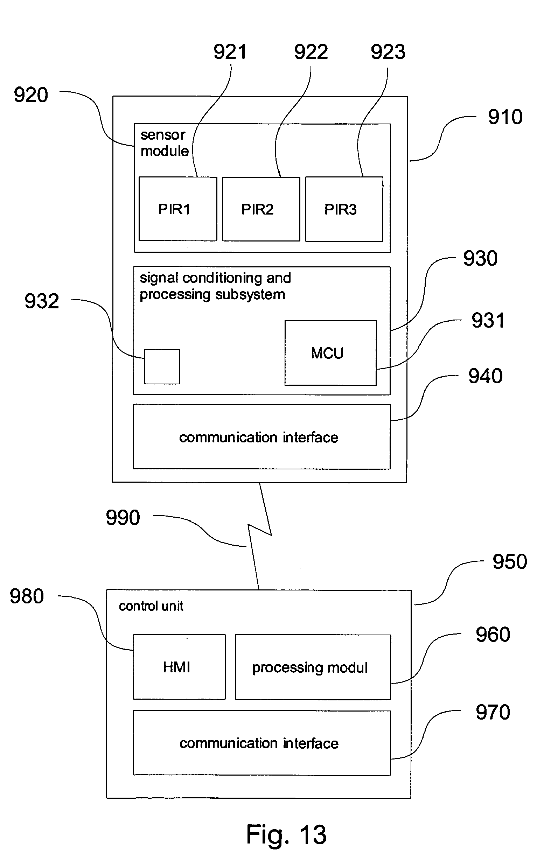

**Fig. 13** shows an embodiment of a complex monitoring system. The MCU **931** is configured that way that exceeding an above mentioned threshold level generates an alert signal. The alert signal may be transmitted to a control unit **950,** where the data might be further processed in the processing module **960,** and/or the information about the fall might be sent through a communication interface **970** to caregiver personals. Communication interface **970** communicates through a communications connection such as the telephone, GSM, Internet or some other network.

The detection of time period while the person is lying on the floor and the time of getting up can provide valuable information for the caregiver about the condition of the monitored person. With both the 2 and 3 sensors configurations **60** and **70,** the MCU **931** may be configured that way that it counts the elapsed time between the fall and the first detection of upward motion, which indicates an attempt to stand up again. Moreover MCU **931** may be configured to count the elapsed time between the first detection of the upward motion and a horizontal motion, which's time range exceeds a certain threshold level, which would indicate that the person is already in a standing position, and trying to walk. This information may be sent to the control unit **950** for further processing and/or to a device where this information is presented to the caregiver.

Furniture might play a role in the reliability of the detection, however placing more sensors in the room can help to achieve a more error prone fall detection system. With regard to the furniture in the room, the following aspects can be taken into consideration: With 2 and 3 sensor configurations **60, 70,** in case the sensor has a view on the front side of the sofa or other seating, and the person can be detected in lying position, the MCU may be configured to be able to differentiate between the fall and lying down by taking the following parameters **932** into consideration: duration of lower sensor signal detection is shorter downwards, and most probably will contain an upwards motion component as well, when the person rises his leg on the sofa. It is probable to have a waiting period between sitting and lying position, thus if a "sitting down" event is followed by a motion downwards, than there is a high probability that no fall event occurred. Between sitting and lying position a smaller amount of horizontal motion can be detected, since only the head is visible by the horizontal sensor, while a full body fall have a higher probability to contain larger horizontal components.

Referring to **Fig. 13****,** the PIR fall detection device **910** of the fall detection system is shown in a block diagram with a control unit **950.** Various number of PIR fall detection devices **910** of the fall detection system are configured to be mounted in different locations of the patient's living area such as the living room, bedroom, bathroom, kitchen and other locations in the home. The PIR fall detection devices **910** communicate with the control unit **950.** The communication channel **990** may be wired or wireless. The console of the control unit **950** is typically located in the living area of the monitored person, where it is convenient for the patient to place and handle it. The control unit **950** may communicate with a central server that may provide administration for the system and passes alerts to caregiver terminals. The PIR fall detection devices **910** of the fall detection system includes the communication interface **940,** the sensor module **920** having at least two sensors **921, 922, 923,** the signal conditioning and processing subsystem **930** with at least one parameter **932.**

Alternatively there are many arrangements are possible compared to the above described arrangement. There can be other arrangements such as in which the control unit **950** communicates with the caregiver terminal, or directly with caregivers, either through a communications connection such as the Internet, telephone, 3G, GSM or any wired or wireless networks.

Several embodiments of the user interface **980** are possible depending on the specific needs of the user installation. For home-based monitoring, an example user interface **980** takes the form of a PC with touch screen or other human machine interface that is located in the patient's house. The user interface might be in the same housing with the control unit, or alternatively might be in a separated housing connected with wired or wireless communication network.

In another embodiment user interface **980** comprises an announcement device and voice recognition capabilities to perform communication with the patient without the patient physically interacting with the consol, and without caregiver involvement in the initial communication.

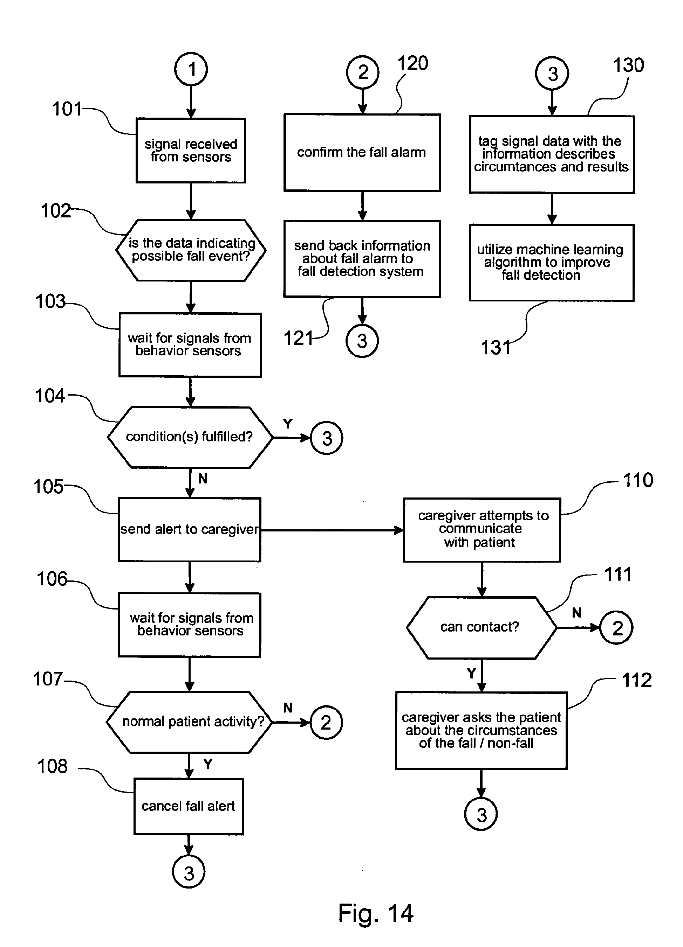

In one possible corresponding flow-chart of fall detection is presented in **Fig. 14****.** Once the PIR fall detection device **910** receives signals from the sensors in step **101,** a detection of a possible fall event is to be decided at step **102.** Then the system waits for signals from behavior sensors in step **103.** All the related information, including parameters **932** of the processing algorithms, will be sent to the control unit **950,** to storing and further analysis. The control unit **950** has a processing module **960,** which is configured to make decision based on the incoming data, and in case the data fulfills the predetermined conditions, as compared in step **104,** which will indicate a real fall event, alert signal is sent to the caregiver in step **105.** Conditions are for example allowing fall alert cancelling or not, multiple residents are in the house, and so on.

The above mentioned behavior sensors are performing continuous measurement of behavioral data of the individuals to be monitored. Such data can be for example but not exclusively determining the motion and/or location of the individual in a selected area, and/or a body-worn sensor for sensing the activity such as speed and/or acceleration of the motion of a selected body part of the individual for determining the motion activity of the individual.

Once caregiver receives an alert, he or she will attempt to communicate with the patient in step **110** at the monitored site to verify that there is no medical or emergency issue. If the caregiver cannot contact to the patient in step **111,** or patient confirms the fall, alarm is raised in step **120.** At step **121** alarm information is sent back to the fall detection system, which is configured to tag the data in step **130** related to alarm for further processing. The caregiver alternatively may ask the patient about the circumstances of the fall/non-fall event in step **112.**

System may be configured to cancel a fall alert in step **108** once any kind of activity is detected from the patient at step **106,** which activity indicates that patient is performing normal daily activity as recognized in step **107.**

One of the most common way to utilize the PIR fall detection device **910** is to use it in a fall detection system, where the alerts generated by the sensor can be processed by the processing module **960** in the control unit **950,** which collects and process the data arriving from many other PIR fall detection devices **910** utilized in the system. In a fall detection system where a sensor network is available the system may be configured to cancel a fall alert **108** if after the detected fall event motion data are received from other sensor locations, which indicates a possible false positive detection. Alternatively any other device, which is able to record activity from the patient could provide with this information. If fall alert cancellation **108** is activated in the system on one hand the patient need to live alone, or the system must be ready to handle pets and multiple residents in the patient's home.

Pet immunity may be achieved in the PIR fall detection device with proper algorithms that are designed for this purpose.

The presence of multiple persons in the monitored area can be detected either with this system or with other conventional motion detection systems. System may be configured that in case of a fall event is detected system warns the other person within the home to help.

Furthermore the fall detection system may comprise pressure sensors placed on chairs and sofa which provides further information about the position of the person, thus making possible to ignore false fall events in case of sitting or lying down on a furniture where pressure sensor is present. On the other hand inserting additional sensors in the system makes the system more expensive.

In a fall detection system the PIR fall detection device **910** might be extended with reinforcement learning capability, thus allow the system to learn abnormal behavior of the patient. It may be achieved in the following way: when the PIR fall detection device **910** is detecting a fall, the information is sent to the control unit **950,** which comprises a user interface **980** (human machine interface) to communicate with the patient, and which is able to ask the patient to confirm the fall event. Alternatively the user interface **980** might be placed in a different location from the control unit **950,** but connected with any appropriate communication method. After the confirmation of the fall event, the system sends back the data representing the answer of the person to the PIR fall detection device **910,** which stores the pattern of the detected event and label it with fall or no fall information. The MCU may be configured to run a machine-learning algorithm in step **131** (clustering algorithm, like k-means algorithm) that utilizes the tagged information to update the model used by the fall detection algorithm.

In one embodiment the system is configured to gain information from the person about the possible fall event. In possible one arrangement the fall event can be confirmed with a user interface **980** (terminal) placed in the patient's house. In this case, if there is no answer within a predefined time period, the system infers that a fall event have occurred with high probability where the person needs help of care providers. If the person is able to get up, and goes to the user interface **980** (terminal) there are 2 choices indicated (e.g. on a screen) to choose from: the fall can be confirmed or denied. In case the fall event has been denied by the patient a machine learning algorithm will be trained with the negative example, in order to avoid generating alert for the same case in the next time, otherwise the learning algorithm stores the features of this event as a positive example, and this data can be utilized as training data as well.

In another embodiment the system may be configured to perform the fall event confirmation via voice message. This case the person is informed with a voice message that a possible fall have been detected, and the patient's answer is detected with voice recognition, where the patient can either confirm or deny the fall event. The patient's voice is detected with a microphone and processed with a speech recognition algorithm as part of the fall detection system.

There are several features that the algorithm of the PIR fall detection device **910** on the MCU **931** can take into consideration. Feature can be the frequency components of signal from the sensors, the phase of the signals, which indicate direction, the timing of the signals, where the order of the signals are taken into account, the time difference between the signals from the different sensors, the elapsed time after the last signal have been received, and in case analog digital converter is utilized instead of the comparators, the amplitude of the signal can be taken into account as well.

With the previously presented invention a very cheep device can be provided and largely accepted system can be built in any in-house environment which is able to reliably detect fall of an elderly person in the field of view of the detector.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. Device for providing fall detection to be used in a health monitoring system in an in-house room environment, the device comprising
- a PIR (Passive Infra Red) detection unit **(10),** having a given field of view **(3),** and mounted on a vertical surface, the PIR detection unit **(10)** comprising
- a dual element PIR sensor **(12, 14)** having an axis of symmetry,
- an infrared lens placed in front of the PIR sensor **(12, 14),**
where the infrared lens divides a plane orthogonal to the axis of symmetry into a plurality of zones **(1)** in the given field of view **(3)** of the PIR detection unit **(10),** each zone **(1)** comprising two different sectors **(2),**
**characterized in that** the infrared lens is a Fresnel lens array **(11, 13)** having an optical focusing effect in a direction parallel with the axis of symmetry of the corresponding dual element PIR sensor **(12, 14);** the two dual element PIR sensors **(12, 14)** have orthogonal axis of symmetry; and an output signal of both dual element PIR sensors **(12, 14)** are forwarded to a signal processing MCU **(35)** for evaluation of a phase of the output signals of the dual element PIR sensors **(12, 14)** and producing a fall detection alert signal in response to evaluation by the signal processing MCU **(35).**

2. The device of claim 1, in which the different dual element PIR sensors **(12, 14)** of the different PIR detection unit **(10)** comprises a horizontal PIR sensor **(12)** having vertical axis of symmetry, and vertical PIR sensor **(14)** having horizontal axis of symmetry.

3. The device of claim 2, in which the PIR detection unit having vertical axis of symmetry comprises Fresnel lens array **(11)** dividing the plane orthogonal to the axis of symmetry into several horizontal zones **(1)** in the field of view **(3)** of the corresponding PIR sensor **(12).**

4. The device of claim 2, in which the PIR detection unit **(10)** has horizontal PIR sensor **(12)** with corresponding Fresnel lens array **(11)** having a zone **(4)** narrower than 10°.

5. The device of claim 1, in which the output signals of the PIR sensors **(30, 31)** are forwarded to the MCU **(35)** through amplifiers **(32)** and subsequent corresponding positive signal comparators **(33)** and negative signal comparators **(34).**

6. The device of claim 1, in which the output signals of the PIR sensors **(40, 41)** are forwarded to the MCU **(45)** through band pass filters **(42, 43).**

7. The device of claim 2, in which the PIR detection unit **(10)** is mounted on a vertical surface at a position in which the vertical PIR sensor **(14)** is in 0.4 - 0.9 m distance from the floor.

8. The device of claim 1, in which an additional vertical movement detecting second vertical PIR sensor **(52, 77)** is placed at a vertical distance from the first vertical movements detecting PIR sensor **(51, 76)** of the PIR detection unit **(10).**

9. The device of claim 8, in which the second vertical PIR sensor **(52, 77)** is arranged within the PIR detection unit **(10).**

10. A Method for providing a fall detection signal to be used in a health monitoring system in an in-house room environment, the method comprising the steps of
- placing an infrared Fresnel lens array **(11, 13)** in front of a first and a second dual element PIR sensors **(12, 14),** which Fresnel lens array **(11, 13)** has optical focusing effect in a direction parallel with an axis of symmetry of one of the first and second dual element PIR sensors **(12, 14);**
- applying a PIR detection unit **(10),** comprising the first and second dual element PIR sensors **(12, 14)** of an orthogonal axis of symmetry relative to one another;
- forwarding an output signal of both first and second dual element PIR sensors **(12, 14)** to a signal processing MCU **(35);** and
- evaluating a phase of the output signal of both the first and second dual element PIR sensors **(12, 14)** and
- producing a fall detection alert signal in response to a result of said evaluating step.

11. The method of claim 10, in which the output signals of the PIR sensors **(30, 31)** are forwarded to the MCU **(35)** through amplifiers **(32)** and subsequent corresponding positive signal comparators **(33)** and negative signal comparators **(34),** and the evaluating is carried out by counter phase detecting within a predetermined time frame.

12. The method of claim 11, in which the predetermined time frame is an adjustable value between 1 to 30 seconds.

13. The method of claim 12, in which the fall is detected when a detection of an up to down movement is not followed by a down to up or a horizontal movement detection in said predetermined time frame.

14. The method of claim 10, in which an additional vertical movement detecting second vertical PIR sensor **(77)** is applied, which is placed at a vertical distance from the first vertical movements detecting PIR sensor **(76)** of the PIR detection unit **(10),** and the output signals of the first vertical PIR sensor **(76)** and the second vertical PIR sensor **(77)** are used together for evaluating and producing fall detection alert signal in response to the result of said evaluation.

15. A system comprising at least one or a plurality of devices for providing fall detection, which device comprising
- a PIR (Passive Infra Red) detection unit **(10),** having a given field of view (3), and mounted on a vertical surface, the PIR detection unit **(10)** comprising
- a dual element PIR sensor **(12, 14)** having an axis of symmetry,
- an infrared lens placed in front of the PIR sensor **(12, 14),**
where the infrared lens divides a plane orthogonal to the axis of symmetry into a plurality of zones **(1)** in the given field of view **(3)** of the PIR detection unit **(10),** each zone **(1)** comprising two different sectors **(2),**
**characterized in that** the infrared lens is a Fresnel lens array **(11, 13)** having an optical focusing effect in a direction parallel with the axis of symmetry of the corresponding dual element PIR sensor **(12, 14);** the two dual element PIR sensors **(12, 14)** have orthogonal axis of symmetry; and an output signal of both dual element PIR sensors **(12, 14)** are forwarded to a signal processing MCU **(35)** for evaluation of a phase of the output signals of the dual element PIR sensors **(12, 14)** and producing a fall detection alert signal in response to evaluation by the signal processing MCU **(35),** and in which system a control unit **(950)** comprising a user interface **(980)** is connected to the at least one said device through communication interfaces **(940),** and the control unit **(950)** is capable to establish real-time communication between a caregiver person and a person being in a supervised in-house room environment.
